# EUROPÄISCHE PATENTANMELDUNG

(11) **EP 0 582 063 A1**
(43) Veröffentlichungstag der Anmeldung: **09.02.1994**
(21) Anmeldenummer: 93109095.5
(22) Anmeldetag: 07.06.1993
(51) Int. Cl.: C07D 213/06, C07C 67/38, B01J 31/02, C07B 41/12

(54) **Verfahren zur Reingewinnung von Pyridin-Verbindungen aus Hydrocarboxymethylierungsgemischen**

(30) Priorität: 06.08.1992 DE 4225992
(71) Anmelder: HÜLS AKTIENGESELLSCHAFT, D-45764 Marl (DE)
(72) Erfinder: Sridhar, Srinivasan, Dr., D-4370 Marl (DE); Hartmann, Manfred, D-4370 Marl (DE)

(57) **Zusammenfassung**

Zur Reingewinnung von Pyridin-Verbindungen wird den Hydrocarboxymethylierungsgemischen zunächst Wasser zugesetzt. Anschließend werden Methanol, Zwischensieder und dann die Pyridin-Verbindungen abdestilliert. Dabei werden die Pyridin-Verbindungen bei vermindertem Aufwand in hoher Reinheit erhalten.

## Beschreibung

Die Erfindung betrifft ein neues Verfahren zur Reingewinnung von Pyridin-Verbindungen aus Gemischen, die bei der Hydrocarboxymethylierung von 6 bis 14 C-Olefinen erhalten werden.

Carbonsäuren können in Form ihrer Methylester hergestellt werden, indem man Olefine katalytisch mit Methanol und Kohlenmonoxid umsetzt. Diese als Hydrocarboxymethylierung bezeichnete Reaktion ist in der Literatur bekannt. Aus C₆- bis C₁₄-Olefinen können unverzweigte Fettsäureester mit 7 bis 15 C-Atomen gewonnen werden. Zur Herstellung wird meist ein Katalysatorsystem verwendet, das neben einer Co-Verbindung, wie z. B. Co-Naphthenat, auch ein Picolin enthält. Nach der Reaktion soll das Produkt nach Möglichkeit in folgende Ströme aufgefächert werden:
1. Methanol: Rückführung des nicht abreagierten Edukts für die Reaktion
2. Picolin: Rückführung zum Katalysatoransatz
3. Co-Katalysator und Produktströme
Das Erreichen dieser Ziele wird dadurch erschwert, daß die zurückzuführenden Stoffe nicht nur einen weiten Siedebereich (von Methanol bis zum Co-Rückstand) aufweisen, sondern auch von einer Vielzahl von unerwünschten Zwischensiedern begleitet werden. Insbesondere wird die Abtrennung von Picolin von denjenigen Zwischensiedern erschwert, die im Siedebereich vor und nach Picolin sieden. Es handelt sich um Kohlenwasserstoffe sowie um weitere zahlreiche Komponenten, die nicht im einzelnen identifiziert sind.

In Ind. Eng. Chem. Prod. Res. Dev. 1980, 19, 330 - 334, wird die Hydrocarboxymethylierung mit anderen Methoden zur Herstellung von Estern verglichen. Danach ist bei der Hydrocarboxymethylierung wegen der Gegenwart von Methanol und größeren Mengen Promotor ein vergleichsweise hoher Destillationsaufwand zur Gewinnung der Produkte erforderlich.

In Hydrocarbon Processing 60 (Okt. 1981), 151 - 57, wird bei der Hydrocarboxymethylierung für die Aufarbeitung des Reaktionsgemisches eine Anordnung mit 5 Destillationskolonnen angegeben. Am Kopf der ersten 3 Kolonnen werden danach Leichtsieder, Methanol und Promotor (Pyridin) destilliert. Dabei ist zur Abtrennung des Promotors eine Säule mit hoher Trennleistung erforderlich.

Wir haben 1,7-Octadien mit Hilfe von Co-Naphthenat/4-Picolin hydrocarboxymethyliert und dann das Reaktionsprodukt nach Hydrocarbon Processing 60, Seite 155, aufgetrennt. Nach Destillation von Leichtsiedern und Methanol wurde das Sumpfprodukt der 1. Kolonne auf eine leistungsfähige Kolonne mit 80 Glockenböden und DN 80 (Nennweite) gegeben, um 4-Picolin abzutrennen. Bei einer Kopftemperatur von 75 °C/100 hPa und einer Sumpftemperatur von 132 °C/230 hPa und trotz eines hohen Rücklaufverhältnisses von 20 bestand das Destillat nur zu 92 bis 93 % aus 4-Picolin. Weitere Produkte waren nicht nur Hochsieder, sondern auch Leichtsieder (Sdp. 138 bis 144 °C) und Mittelsieder (Sdp. 144 bis 177 °C), verglichen mit 4-Picolin.

Daraus geht hervor, daß die Begleitkomponenten von 4-Picolin nicht leicht abzutrennen sind und daß auch bei einer Erhöhung des Trennaufwandes, beispielsweise durch eine Verlängerung des Verstärkerteils der Kolonne oder durch ein noch höheres Rücklaufverhältnis, ein Destillat mit einer beträchtlichen Konzentration an Begleitkomponenten zu erwarten ist. Verluste an 4-Picolin sind unvermeidlich.

Es bestand daher die Aufgabe, bei der Reingewinnung von Pyridin-Verbindungen den Destillationsaufwand zu verringern und die Reinheit der Produkte zu erhöhen.

Die Aufgabe wird erfindungsgemäß dadurch gelöst, daß man dem Hydrocarboxymethylierungsgemisch Wasser zusetzt und dann Methanol, Zwischensieder mit Hilfe von Wasser und danach die Pyridin-Verbindungen destillativ abtrennt.

Überraschenderweise vereinfacht die Wasserzugabe die Destillation der Pyridin-Verbindungen erheblich.

Geeignete Pyridin-Verbindungen sind Pyridin, 3- und 4-Picolin, 3,4- und 3,5-Lutidin sowie 3- und 4-Ethylpyridin. 3- und 4-Picolin werden vorzugsweise bei der Hydrocarboxymethylierung eingesetzt, wobei 4-Picolin ganz besonders bevorzugt wird.

Für die Hydrocarboxymethylierung können beispielsweise Olefine wie 1-Hexen, 1-Hepten, 1-Octen, 4-Octen, 1-Nonen, 1-Decen oder 1-Dodecen eingesetzt werden. Man kann auch Diene wie 1,5-Hexadien, 1,7-Octadien, 1,9-Decadien und 1,11-Dodecadien verwenden. Außerdem können auch verzweigte Olefine hydrocarboxymethyliert werden. Die Olefine weisen vorzugsweise 8 bis 12 C-Atome auf.

Vorzugsweise werden mindestens 0,01 Teile Wasser pro Teil Pyridin-Verbindung zugesetzt. Dabei liegt der Gehalt im allgemeinen bei 0,01 bis 2 Teilen Wasser und im besonderen bei 0,05 bis 1 Teil Wasser, jeweils bezogen auf die Pyridin-Verbindung.

Die destillative Auftrennung des Hydrocarboxymethylierungsgemisches kann ansatzweise und koninuierlich durchgeführt werden.

In einer besonderen Ausführungsform wird Wasser zugesetzt, das bei der nachfolgenden destillativen Abtrennung von Methanol, Zwischensieder und Pyridin-Verbindung im Kreise geführt wird. Dabei wird ein Wassergehalt von 0,01 bis 2 Teilen pro Teil Pyridin-Verbindung aufrechterhalten.

Nach dem vorliegenden Verfahren wird die Pyridin-Verbindung bei vermindertem Destillationsaufwand in > 99%iger Reinheit gewonnen. Dabei sind die Verluste an dieser Verbindung sehr gering. Unerwünschte Mitsieder werden nach der vorliegenden Erfindung praktisch vollständig gesondert abgetrennt.

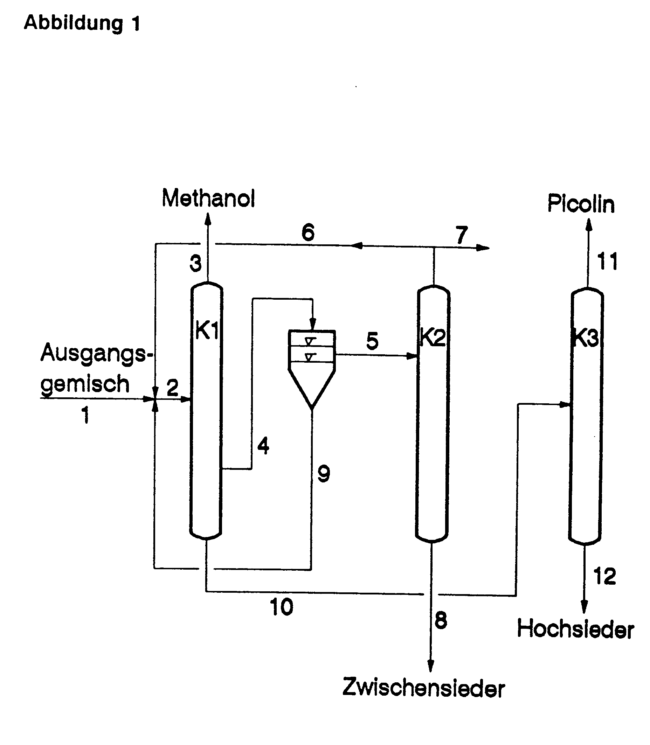

Abbildung 1 stellt eine bevorzugte Anordnung dar, in der die Erfindung ausgeführt werden kann:
Hydrocarboxymethylierungsgemisch wird zusammen mit dem zugesetzten Wasser auf die Kolonne K 1 gegeben. Methanol wird am Kopf dieser Kolonne destilliert. Als Seitenabzug entnimmt man ein Azeotrop, wobei man nach Abkühlung und Phasentrennung die wäßrige Phase zurückführt, während man die organische Phase als Strom 5 in die Kolonne K 2 leitet. Dort werden Zwischensieder als Sumpfprodukt abgetrennt. Das Kopfprodukt, die Pyridin-Verbindung, wird dagegen auf die Kolonne K 1 zurückgeführt. Mit der Pyridin-Verbindung siedende Komponenten können über Strom 7 ausgeschleust werden und so dem Kreislauf der Ströme 2, 4, 5 und 6 entzogen werden. Das Sumpfprodukt der Kolonne K 1 wird als Strom 10 auf die Kolonne K 3 gegeben, wo die Pyridin-Verbindung, beispielsweise Picolin, am Kopf abdestilliert wird.

Vorzugsweise erfolgt die Phasentrennung des Azeotrops von Strom 4 bei 10 bis 90 °C und Normaldruck. Die Kolonne K 2 wird vorzugsweise bei einem Druck von 30 bis 1 020 hPa betrieben.

### Beispiel

Dem Reaktionsgemisch, das bei der Hydrocarboxymethylierung von 1,7-Octadien mit Methanol und CO unter Katalyse von Co-Naphthenat/4-Picolin anfällt, werden pro Teil eingesetztem 4-Picolin 1 Teil Wasser zugemischt. Dann benutzt man eine Kolonnenanordnung gemäß Abbildung 1.

In K 1 wird als Destillat Methanol und der mit Methanol azeotrope Teil der Kohlenwasserstoffe abgetrennt. Die übrigen Kohlenwasserstoffe (Zwischensieder) werden im Abtriebsteil von K 1 über Strom 4 abgeführt. Strom 4 enthält dabei eine Mischung aus Kohlenwasserstoff/Wasser/4-Picolin-Azeotrop und überschüssigem 4-Picolin. Nach Phasentrennung wird 4-Picolin enthaltendes Wasser zurückgeführt. Die organische Phase, die noch 7 % 4-Picolin enthält, wird in die Stripkolonne K 2 geleitet. Hier wird das Destillat, ein 4-Picolin-Azeotrop, nach K 1 zurückgeführt. Die Zwischensieder werden aus dem Sumpf von K 2 ausgetragen.

Das wasserfreie Sumpfprodukt von K 1 wird auf die Kolonne K 3, die 60 Glockenböden und DN 80 aufweist, geleitet. Bei einem Rücklaufverhältnis von 10 wird > 99%iges 4-Picolin am Kopf destilliert. Das Sumpfprodukt ist frei von 4-Picolin und enthält hochsiedende Komponenten.

## Patentansprüche

1. Verfahren zur Reingewinnung von Pyridin-Verbindungen aus Gemischen, die bei der Hydrocarboxymethylierung von 6 bis 14 C-Olefinen erhalten werden,
dadurch gekennzeichnet,
daß man Wasser zusetzt und dann Methanol, danach Zwischensieder mit Hilfe von Wasser und schließlich die Pyridin-Verbindungen destillativ abtrennt.

2. Verfahren nach Anspruch 1,
dadurch gekennzeichnet,
daß die Pyridin-Verbindungen 3- und 4-Picolin sind.

3. Verfahren nach Anspruch 2,
dadurch gekennzeichnet,
daß 4-Picolin gewonnen wird.

4. Verfahren nach Anspruch 1,
dadurch gekennzeichnet,
daß die Olefine 8 bis 12 C-Atome aufweisen.

5. Verfahren nach Anspruch 1,
dadurch gekennzeichnet,
daß man mindestens 0,01 Teil Wasser pro Teil Pyridin-Verbindung zusetzt.

6. Verfahren nach Anspruch 5,
dadurch gekennzeichnet,
daß man 0,01 bis 2 Teile Wasser pro Teil Pyridin-Verbindung zugibt.

7. Verfahren nach Anspruch 6,
dadurch gekennzeichnet,
daß man 0,05 bis 1 Teil Wasser pro Teil Pyridin-Verbindung zufügt.

8. Verfahren nach Anspruch 1,
dadurch gekennzeichnet,
daß man Wasser zusetzt, es bei der Destillation von Methanol, Zwischensieder und Pyridin-Verbindung im Kreise führt und dabei einen Gehalt von 0,01 bis 2 Teilen Wasser pro Teil Pyridin-Verbindung einstellt.

9. Verfahren nach Anspruch 1,
dadurch gekennzeichnet,
daß man gemäß Abbildung 1 Methanol am Kopf der Kolonne K 1 entnimmt, als Seitenabzug ein Azeotrop entnimmt, nach Phasentrennung die wäßrige Phase zurückführt und die organische Phase in die Kolonne K 2 leitet, wo man Zwischensieder als Sumpfprodukt abtrennt und das Kopfprodukt in die Kolonne K 1 zurückführt, das Sumpfprodukt der Kolonne K 1 auf die Kolonne K 3 gibt und dort reine Pyridin-Verbindung als Kopfprodukt gewinnt.
